## Europäisches Patentamt
## European Patent Office
## Office européen des brevets

(11) Publication number: **0 142 889**
**B1**

(12) **EUROPEAN PATENT SPECIFICATION**

(45) Date of publication of patent specification: **16.08.89**

(51) Int. Cl.⁴: **C 07 C 1/04,** C 10 J 3/54,
C 10 G 11/18, C 10 G 9/32

(21) Application number: **84201626.3**

(22) Date of filing: **09.11.84**

(54) Process for the preparation of hydrocarbons and a fuel gas.

(30) Priority: **16.11.83 GB 8330606**

(43) Date of publication of application:
**29.05.85 Bulletin 85/22**

(45) Publication of the grant of the patent:
**16.08.89 Bulletin 89/33**

(84) Designated Contracting States:
**BE DE FR GB IT NL**

(56) References cited:
**EP-A-0 101 878**
**GB-A- 671 490**
**GB-A-2 077 289**
**US-A-2 436 938**
**US-A-4 050 908**
**US-A-4 186 079**

(73) Proprietor: **SHELL INTERNATIONALE
RESEARCH MAATSCHAPPIJ B.V.**
**Carel van Bylandtlaan 30**
**NL-2596 HR Den Haag (NL)**

(72) Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a process for the preparation of hydrocarbons and fuel gas from a heavy carbonaceous feedstock by isolating in a first step a part of the hydrocarbons from the feed, converting in a second step the remaining part of the feed into synthesis gas, and converting in a third step synthesis gas into hydrocarbons.

The preparation of hydrocarbons from a heavy carbonaceous feedstock is known from U.S. Patent 2,436,938. In this patent a distillation-extraction treatment of solid carbonaceous materials containing volatile hydrocarbon substituents is described. Examples of the solid carbonaceous materials are coal, lignite and oil shale. The residue of the distillation-extraction treatment is reacted with steam and oxygen to produce water gas. This gas mixture is used as feed for the catalytic synthesis of liquid hydrocarbons. Optionally, a fraction of the thus prepared liquid hydrocarbons may be used for the distillation-extraction treatment. By extraction of the solid carbonaceous materials containing volatile hydrocarbon constituents with a hydrocarbon oil being essentially free of cyclic hydrocarbons, the volatile hydrocarbons are removed, while the pitch and tars substantially remain in the solid carbonaceous materials. The extract may be processed in the usual way, and the extracted solid carbonaceous materials are subjected to steam reforming to produce water gas, which is used for the synthesis of liquid hydrocarbons.

In U.S. Patent 4,186,079 the preparation of hydrocarbons from a heavy carbonaceous feedstock is described. The carbonaceous feed and a particulate heat carrier at a temperature between 855 and 1095°C are contacted in a pyrolysis zone. The light hydrocarbons evaporate and are separated from the heat carrier. The heavy hydrocarbons ("coke") are deposited on the heat carrier and are partially combusted in a combustion zone. The heated carrier is mixed in a synthesis gas generation zone with steam to produce synthesis gas and heat carrier at a suitable temperature for introduction into the pyrolysis zone.

The present invention, now, relates to a thermal cracking process of a heavy hydrocarbonaceous feedstock producing hydrocarbons and coke. This cracking process is carried out in the presence of fluidized solids on which the coke is deposited. The coked fluidized solids are gasified with an oxygen containing gas to produce synthesis gas. This gas is used for the catalytic synthesis of liquid hydrocarbons. The unreacted gases (CO and $H_2$) are together with gaseous hydrocarbons used as fuel.

The catalytic synthesis of hydrocarbons in the present invention is carried out in such a way that the mixture of carbon monoxide and hydrogen is only partially converted into hydrocarbons. In the coke gasification process large amounts of low BTU fuel gas are produced. This gas may be combusted in furnaces close to the process plant.

However, operation on a commercial scale involves a too great production of this low value heating gas containing at least CO, $H_2$, $N_2$, $CO_2$ and $H_2O$. Transport of the gas over larger distances involves too high expenses. Therefore, this low value heating gas is partially converted into hydrocarbons. By means of this partial conversion an amount of normally liquid hydrocarbons is produced which may be used as a valuable fuel or as a chemical feedstock. The volume of the remaining part of the unconverted mixture of carbon monoxide, hydrogen, nitrogen, carbon dioxide etc., together with the produced normally gaseous hydrocarbons is of course substantially smaller than the volume of the starting mixture.

It could be expected that the decreased amount of carbon monoxide and hydrogen in the off-gas would involve a proportional decrease of the heating value. The high heating value of the normally gaseous hydrocarbons present in the off-gas and the decreased volume, however, warrants that the off-gas as a whole has a heating value comparable to or not much lower than that of the carbon monoxide- and hydrogen-containing gas mixture used for the conversion. Further, a considerable quantity of liquid hydrocarbons is produced. Thus, by the above partial conversion a reduction in the quantity of the low heating value gas is obtained, while the quality of the remaining low heating value gas is still at a sufficient level, and valuable liquid hydrocarbons are obtained in a considerable quantity.

The present invention relates to a process for the preparation of hydrocarbons and fuel gas from a heavy carbonaceous feedstock by isolating in a first step a part of the hydrocarbons from the feed, converting in a second step the remaining part of the feed into synthesis gas, and converting in a third step synthesis gas into hydrocarbons, characterized in that the heavy carbonaceous feedstock is cracked in a coking zone which contains a bed of fluidized solids producing hydrocarbons and coke which deposits upon the fluidized solids, introducing a part of the coke-containing solids into a gasification zone in which at least a part of the coke is gasified with an oxygen-containing gas yielding a carbon monoxide- and hydrogen-containing gas mixture, converting the carbon monoxide- and hydrogen-containing gas mixture for 30 to 70% vol, calculated on the volume of carbon monoxide and hydrogen, into hydrocarbons in a synthesis zone, and separating a fuel gas comprising carbon monoxide, hydrogen and gaseous hydrocarbons, and at least one liquid hydrocarbon fraction from the product of the synthesis zone.

In the coking zone a carbonaceous feedstock is cracked in a fluid bed operation. Many kinds of feedstocks may be applied, like heavy hydrocarbon oils such as long or short residues, pitch, asphalt, bitumen, tar, shale oil, coal liquefaction products, but also solid fuels like coal or slurries of coal in oil and/or water. In the coking zone hydrocarbons evaporate and remaining coke deposits upon the fluidized solids. The solids may be any solids which can resist the process conditions, like sand, silica or alumina particles. Preferably the solids are coke particles. Their size suitably is 40—1000 microns. This way no inerts are to be handled in the present process. The particles are fluidized by means of a fluidizing gas which enters the coking zone near or through the bottom. The fluidizing gas, preferably, is steam. The feedstock which is preferably preheated to a temperature of 250—400°C, may be introduced into the fluidized bed through one or more nozzles.

The process is suitably a thermal cracking process carried out at a temperature between 425 and 700°C and a pressure between 1 and 15 bar. The heat to maintain the coking temperature is provided by hot solids, advantageously having a temperature of 50—150°C above the coking temperature, which are recycled from the gasification zone or from a heating zone to be discussed hereafter. The steam preferably used as fluidizing gas may be preheated too, so that it adds heat to the coking zone or does not take up too much heat from it. Suitably the fluidizing gas, such as steam, has a temperature ranging from 400 to 600°C.

In the coking zone the feedstock yields hydrocarbons with various chain lengths. The hydrocarbons produced are preferably split up in at least one gaseous fraction and at least one liquid fraction. One may split the product of the coking zone into a gaseous fraction containing methane, ethane and ethylene, and another gaseous fraction comprising the $C_3$ and $C_4$ hydrocarbons. The latter fraction may be used as liquified petroleum gas (LPG). The products containing 5 or more carbon atoms per molecule, which products suitably are recovered as the liquid hydrocarbon fraction(s), may be split up into a gasoline, a kerosene, a gas oil and a residual fraction.

The coke-containing solids which may consist of coke alone, are preferably continuously discharged from the coking zone, while make up solids are fed into said coking zone. The solids discharged from the coking zone are suitably at a temperature of 425 to 700°C. Before being introduced into the gasification zone the coke-containing solids obtained from the coking zone are preferably heated. Thereto any conventional heating method may be used. Very suitably, the solids are heated in a fluidized bed by means of a hot fluidizing gas and/or hot fluidized solids which act as heat carriers. Advantageously the carbon monoxide- and hydrogen-containing gas mixture obtained in the gasification zone is applied as the hot fluidizing gas, and solids from which the coke at least partially has been converted in the gasification zone are applied as the hot fluidized solids. The coke-containing solids are preferably heated this way to a temperature between 50 and 150°C above the temperature in the coking zone at a pressure between 1 and 15 bar.

After the preferred heating the coke-containing solids are introduced into the gasification zone. The solids from which part of the coke has been converted, may then be applied as heat carrier in the heating and/or the coking zone. Preferably, the gasification is carried out in a fluidized bed. In the fluidized bed the temperature is regulated such that no melting of the solids occurs. Very suitably the oxygen-containing gas acts as fluidizing gas. The oxygen-containing gas may be air, oxygen-enriched air or oxygen. The oxygen-containing gas may be mixed with steam. Preferably the oxygen-containing gas is air, since this way the costs of an expensive air separation plant are saved.

Steam which is preferably introduced into the gasification zone, may be passed thereto together with and/or apart from the oxygen-containing gas. As to the quantities of steam and oxygen-containing gas being introduced into the gasification zone, they are regulated such that the gasification is carried out at a temperature of 800 to 1200°C and a pressure between 1 and 15 bar. When the solids consist of coke alone, the amounts of air and steam are such, that only a portion of the coke is gasified so that hot coke particles remain. The portion of coke gasified preferably corresponds with 90% or more of the net coke production in the coking zone. In case of inert solids upon which coke is deposited, the amounts of air and steam are advantageously such that over 90% of the coke is converted, yielding hot inert solids.

Very suitably at least a part of the hot solids from which coke has been gasified, are recycled to the coking zone as heat carrier. When there is a heating zone between the coking and the gasification zones the hot solids from the gasification zone are preferably passed to the heating zone and subsequently from the heating zone to the coking zone. Ash which may be formed, is discharged from the gasification reactor.

When the above integrated process of coking and gasification is carried out at a commercial scale, a great quantity of coke is formed and a vast amount of carbon monoxide- and hydrogen-containing gas is produced. The heating value of the latter gas is relatively low since it contains in addition to carbon monoxide and hydrogen and hydrogen sulphide; a relatively high percentage of non combustibles such as carbon dioxide, steam, and nitrogen. Advantageously, the gas is desulphurized and then used as fuel gas in a furnace or a similar apparatus on the refinery in which the integrated process has been incorporated. Operation on commercial scale, however, involves a too great production of this low heating value gas so that not all the gas produced can be usefully combusted in furnaces close to the process plant. Moreover, transport of a part of this gas over long distances usually involves too high expenses.

According to the invention the gas is partially converted into hydrocarbons. By means of the partial conversion a substantial amount of hydrocarbons are produced. The hydrocarbons include normally gaseous and normally liquid hydrocarbons. The normally liquid hydrocarbons are very useful as fuel or chemical feedstock. The normally gaseous hydrocarbons are discharged from the synthesis zone with the off-gas which contains in addition to the normally gaseous hydrocarbons and some carbon monoxide and hydrogen, nitrogen. The high heating value of the normally gaseous hydrocarbons warrant a sufficient heating value of the off-gas. The volume of the off-gas is of course substantially smaller than the volume of the carbon monoxide- and hydrogen-containing gas subjected to the conversion.

For this conversion the carbon monoxide- and hydrogen-containing gas mixture is contacted with a catalyst. Since most heavy carbonaceous feedstocks contain sulphur, the carbon monoxide- and hydrogen-containing gas mixture produced in the gasification zone usually contains some hydrogen sulphide. It is desirable to remove at least most of the hydrogen sulphide as practically all catalysts which are applicable in the conversion are more or less severely poisoned by sulphur compounds. Moreover, in order to avoid environmental pollution fuel gases are advantageously sulphur-free. Therefore, the gas mixture from which fuel gas is produced according to the invention is preferably desulphurized. To desulphurize the gas mixture any conventional desulphurization process may be used. Very suitable are processes in which hydrogen sulphide and optionally part of the carbon dioxide present in the gas mixture are absorbed by an absorbing liquid. Very suitable are the ADIP—and Sulfinol processes, described in British patent Specification Nos. 1,131,989, 972,140, 965,358, 957,260 and 879,090.

The purified remaining gas is preferably contacted with a Fischer-Tropsch catalyst for the production of hydrocarbons from hydrogen and carbon monoxide. Most Fischer-Tropsch catalysts yield hydrocarbons in the range from 1 to over 30 carbon atoms per molecule. Especially the preparation of methane or a $C_1$—$C_4$ hydrocarbon mixture is disadvantageous since in the coking zone considerable amounts of methane and other light hydrocarbons ($C_4^-$) have already been obtained. So an overproduction of these cheap gases should be avoided. Very suitably the conversion is carried out such that a substantial amount of liquid hydrocarbons is prepared. So, preferably a Fischer-Tropsch catalyst is selected, which catalyst is capable of catalysing the conversion of a hydrogen/carbon monoxide gas mixture into liquid hydrocarbons. The catalyst preferably is able to stand considerable amounts of nitrogen and/or carbon dioxide in the hydrogen- and carbon monoxide-containing gas mixture. In said gas mixture 20 to 60% vol of nitrogne and 0 to 10% vol of carbon dioxide may be present.

Therefore, in the synthesis zone the carbon monoxide- and hydrogen-containing gas mixture is contacted preferably with a catalyst comprising cobalt and at least one metal ·selected from ruthenium, zirconium, titanium and chromium on silica, silica-alumina or alumina as carrier. These catalysts are found to be stabilized in the presence of nitrogen and/or carbon dioxide. Moreover, they are excellent catalysts for the conversion of carbon monoxide- and hydrogen-containing gas mixtures into hydrocarbons boiling or or above the kerosene and gas oil range. They are applicable at pressures within a wide range. They are preferably used at pressures from 5 to 50 bar.

The catalyst preferably comprises 3—60 pbw of cobalt and 0.1—100 pbw of ruthenium, zirconium, titanium and/or chromium per 100 pbw of carrier. The catalyst suitably has been prepared by impregnation of the carrier with a solution of one or more compounds of the metal or metals. This includes both a simultaneous impregnation of the carrier by a solution containing all metals involved, and a subsequent deposition of each metal separately by impregnation. It is also very suitable to use a catalyst prepared by kneading the carrier with one or more compounds of the metal or metals in the presence of a liquid. The methods of preparation are well described in British Patent Specification No. 2,077,289 and Netherlands Patent Application Nos. 8204525 and 8203066.

The dependence of the catalyst performance on the cobalt load and the surface area of the catalyst is described in Netherlands Patent Application No. 8301922.

The most preferred catalyst comprises 10—50 pbw of cobalt and 0.1—40 pbw of zirconium on 100 pbw of silica.

The process conditions under the conversion in the synthesis zone is carried out are preferably a temperature between 175 and 300°C, a pressure between 5 and 50 bar and a space velocity from 200 to 2000 Nl · l$^{-1}$ · hr$^{-1}$.

The conversion is a strongly exothermic reaction. Therefore the reaction heat is preferably recovered in the form of steam. This steam may be used as fluidizing gas in the coking zone.

Due to the fact that the gas mixture is converted partially the off-gas of the conversion still contains some carbon monoxide and hydrogen. Even when a catalyst is used which is capable of catalysing the conversion of carbon monoxide and hydrogen into liquid hydrocarbons some gaseous hydrocarbons are inevitably formed. The latter hydrocarbons are discharged with the off-gas together with the unconverted gases and steam formed during the conversion.

One should expect that the decreased amount of carbon monoxide and hydrogen in the off-gas, would involve a proportional decrease of the heating value of the off-gas. The high heating value of the gaseous hydrocarbons and the decreased volume however warrants that the off-gas as a whole has a heating value comparable to or not much lower than that of the carbon monoxide- and hydrogen-containing gas mixture used in the conversion. Further a considerable quantity of liquid hydrocarbons is produced. So, by the above partial conversion a reduction in the quantity of the low heating value gas is achieved while the quality of the remaining low heating value gas is still at a sufficient level, and valuable liquid hydrocarbons are obtained in a considerable quantity.

The heating value of the fuel gas obtained suitably is between 3 and 10 MJ/Nm$^3$. It may be advisable to upgrade the heating value of the off-gas of the synthesis zone when its heat value is rather low, e.g. when the carbon monoxide- and hydrogen-containing gas mixture has been converted almost exclusively into normally liquid hydrocarbons. For this purpose, the fuel gas obtained after separation from the product of the synthesis zone is preferably blended with at least a part of the gaseous hydrocarbons-containing fraction obtained from the product of the coking zone. So, for instance a part of the methane and $C_2$-hydrocarbons produced in the coking zone are added to the fuel gas, the heating value of the gas so obtained being increased thereby.

The liquid hydrocarbon fractions obtained after separation from the product of the synthesis zone are of excellent quality, especially the kerosene and gas oil fractions are excellent. The kerosene fraction comprises especially unbranched hydrocarbon molecules which involves a high smoke point. As the gas oil fraction, too, comprises especially paraffinic hydrocarbons, it possesses a high cetane number. Therefore at least a part of a liquid hydrocarbon fraction obtained after separation from the product of the synthesis zone is preferably blended with at least a part of a liquid hydrocarbon fraction obtained from the product of the coking zone, thereby upgrading the resulting liquid fraction. The fraction boiling above the gasoil range, can be converted to lighter hydrocarbons by catalytic cracking or by hydrocracking. These lighter hydrocarbons may also be blended with appropriate fractions of the coker product.

The invention will now be further illustrated with reference to the figure showing a flow scheme for the process according to the invention, in which figure auxiliary equipment, such as pumps compressors, valves, control devices, is not included. However, the invention is by no means limited to this figure.

A carbonaceous feedstock is fed through a line 2 into a coking reactor 1. Inside the reactor a bed of fluidized solids, e.g. coke particles, is present, which solids are fluidized by means of steam introduced through a line 3. The hydrocarbon products and non-converted steam leave the reactor through a line 4 and are passed to a separator 5 in which the coking product is split up into a gaseous fraction discharged by a line 6, and a liquid fraction discharged by a line 7.

In the reactor 1 fresh coke from the carbonaceous feedstock deposits upon the solids. Coke-containing solids are discharged at the bottom of the reactor through a line 8. They are conveyed to a heater 11 through a line 9 by means of a carrier gas comprising a hot carbon monoxide- and hydrogen-containing gas supplied through a line 10. In the heater 11 a fluidized bed of solids is present. Hot solids are introduced as heat carriers into the heater 11 through a line 14 from a gasification reactor 15. Heated solids are passed from the heater 11 to the coking reactor 1 through a line 13 and from the heater 11 to the gasification reactor 15 through a line 16. The carrier gas from lines 10 and 9 acts as fluidizing gas in the heater 11 and is discharged through a line 12.

In the gasification reactor 15 coke on the solids is partially combusted by means of an oxygen-containing gas such as air and steam, supplied through a line 17, yielding hot solids, a carbon monoxide- and hydrogen-containing gas mixture and ash. The oxygen-containing gas and steam act as fluidizing gas in the gasification reactor 15. The hot carbon monoxide- and hydrogen-containing gas is discharged through the line 10 and passed to the lines 8 and 9. Hot solids are passed to the heater 11 through the line 14 and ash is discharged from the system through a line 18.

The carbon monoxide- and hydrogen-containing gas mixture is passed through the line 12 to a scrubber 19 in which hydrogen sulphide, and optionally $CO_2$ present in the gas, are removed from the gas. The resulting gas is passed through a line 20 into a synthesis reactor 21, loaded with a bed of cobalt- and zirconium-containing silica. The gas mixture is partially converted into hydrocarbons. The product of the synthesis reactor 21 is passed through a line 22 to a separator 23 in which the product is split in a fuel gas, discharged through a line 24 and a liquid fraction discharged through a line 25.

The heating value of the fuel gas in line 24 may be upgraded by blending it with a part of the gaseous product of line 6. Thereto, a part of the latter product is passed from line 6 to line 24 through a line 26. The upgraded fuel gas is discharged through a line 28. The remaining part of the gaseous product of the coking zone is passed for further processing through a line 27.

Example

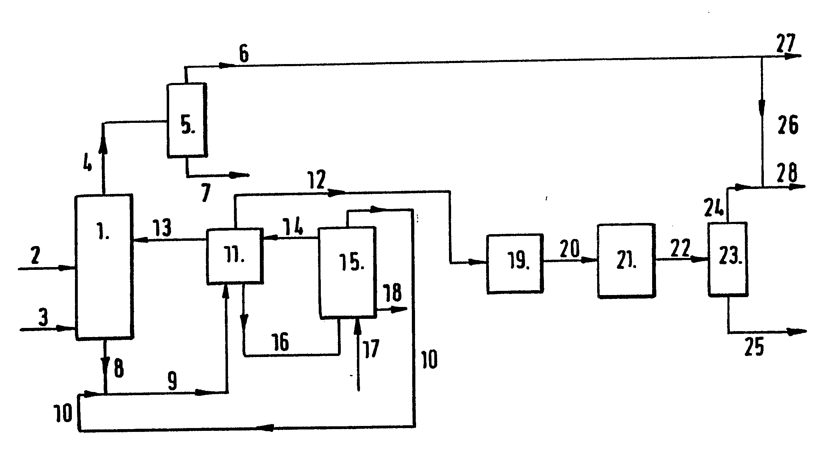

In a process as described in the figure 312 t/hr of a short residue of an Arabian heavy crude oil are passed into a coking reactor in which coke particles are fluidized with steam as a fluidizing gas and in which a temperature of 525°C and a pressure of 2 bar prevailes. The residue has the following properties:

| | | |
|---|---|---|
| Density | kg/m$^3$ | 1050 |
| Sulphur | %w | 6.0 |
| Nitrogen | %w | 0.48 |
| Conradson carbon | %w | 28 |
| Nickel | ppmw | 50 |
| Vanadium | ppmw | 219 |

The rate of residue supply is equivalent to an energy rate of 3390 MW.

The cracking of the residue yields 42.1 t/hr $C_1$—$C_4$ hydrocarbons and 161.3 t/hr liquid $C_5^+$ hydrocarbons boiling below 530°C. Upon the coke particles present in the coking reactor, 109 t/hr of coke are deposited. A steam of coke-containing particles in excess of this amount is discharged from the coking reactor at a temperature of 525°C, heated to 620°C in a heater and passed to a gasification reactor, together with a circulating stream of coke particles. In the gasification reactor 103 t/hr of coke are converted with air and steam into a carbon monoxide- and hydrogen-containing gas. This gas and a circulating stream of hot coke are passed to the heater where they are cooled by the stream of coke particles from the coking zone. The heat released by the hot gas and coke stream from the gasification zone is partly passed to the coking reactor by another stream of coke particles circulating between the heater and the reactor to provide the heat required for the coking reaction.

After condensation of water and removal of acidic components (19.9 t/hr $H_2S$ and 8.5 t/hr $CO_2$) the carbon monoxide- and hydrogen-containing gas is obtained in a quantity of $460 \cdot 10^3$ $Nm^3$/hr and has the following composition:

| | |
|---|---|
| CO | 24.3% vol |
| $H_2$ | 21.3% vol |
| $N_2$ | 51.8% vol |
| Other | 2.6% vol |

The heating value of the gas is 5.7 $MJ/Nm^3$. The gas production represents 730 MW, i.e. 21.6% of the energy introduced with the feed.

If the gas were not subjected to a partial conversion of hydrogen and carbon monoxide into hydrocarbons the above would mean that 21.6% of the feed energy would become available in the form of cheap low BTU fuel gas, in addition to the energy in the form of hydrocarbon gas produced in the coking reactor (13.5%w $C_1$—$C_4$ hydrocarbons) which may also be used as a fuel gas.

According to the invention the purified $H_2$ and $CO_2$ containing gas is contacted with a catalyst comprising 25 pbw of cobalt, 18 pbw of zirconium per 100 pbw of silica, which catalyst has been prepared by impregnation, and which catalyst has a surface area of 100 $m^2$/ml. The conversion is carried out at a temperature of 210°C, a pressure of 20 bar and a space velocity 600 $Nm^3$ $CO+H_2/Nm^3$ cat. hr. Of the CO and $H_2$ in the feed, 55.9% vol. are converted yielding 196 t/hr of liquid $C_5^+$ hydrocarbons and $346 \cdot 10^3$ $Nm^3$/hr of a fuel gas having the following composition:

| | |
|---|---|
| $H_2$ | 5.66% vol |
| CO | 21.09% vol |
| $N_2$+others | 72.12% vol |
| $C_1$—$C_4$ | 1.13% vol |

The heating value of the gas is 4.1 $MJ/Nm^3$. The gas production represents 396 MW, i.e. 11.7% of the feed energy.

The 19.6 t/hr $C_5^+$ hydrocarbons represent 242 MW, i.e. 7.1% of the energy of the feed.

## Claims

1. Process for the preparation of hydrocarbons and fuel gas from a heavy carbonaceous feedstock by isolating in a first step a part of the hydrocarbons from the feed, converting in a second step the remaining part of the feed into synthesis gas, and converting in a third step synthesis gas into hydrocarbons, characterized in that the heavy carbonaceous feedstock is cracked in a coking zone which contains a bed of fluidized solids producing hydrocarbons and coke which deposits upon the fluidized solids, introducing a part of the coke-containing solids into a gasification zone in which at least a part of the coke is gasified with an oxygen-containing gas yielding a carbon monoxide- and hydrogen-containing gas mixture, converting the carbon monoxide- and hydrogen-containing gas mixture for 30 to 70% vol, calculated on the volume of carbon monoxide and hydrogen, into hydrocarbons in a synthesis zone, and separating a fuel gas comprising carbon monoxide, hydrogen and gaseous hydrocarbons, and at least one liquid hydrocarbon fraction from the product of the synthesis zone.

2. Process as claimed in claim 1, in which the cracking process is a thermal process carried out at temperature between 425 and 700°C and a pressure between 1 and 15 bar.

3. Process as claimed in claim 1 or 2, in which the fluidized solids are coke particles.

4. Process as claimed in any one of claims 1—3, in which the solids are fluidized by means of steam as fluidizing gas.

5. Process as claimed in any one of claims 1—4, in which the hydrocarbons produced in the coking zone are split up into at least one gaseous fraction and at least one liquid fraction.

6. Process as claimed in any one of claims 1—5, in which the coke-containing solids obtained from the coking zone are heated before being introduced into the gasification zone.

7. Process as claimed in claim 6, in which the coke-containing solids are heated in a fluidized bed by means of a hot fluidizing gas and/or hot fluidized solids.

8. Process as claimed in claim 7, in which the hot fluidizing gas is the carbon monoxide- and hydrogen-containing gas obtained in the gasification zone, and the hot fluidized solids are solids from which the coke at least partially has been converted in the gasification zone.

9. Process as claimed in any one of claims 6—8, in which the coke-containing solids are heated to a temperature between 50 and 150°C above the temperature of the coking zone at a pressure between 1 and 15 bar.

10. Process as claimed in any one of claims 1—9, in which in the synthesis zone the carbon monoxide- and hydrogen-containing gas mixture is contacted with a catalyst comprising cobalt and at least one metal selected from ruthenium, zirconium, titanium and chromium on silica, alumina or silica-alumina as carrier.

## Patentansprüche

1. Verfahren zur Herstellung von Kohlenwasserstoffen und Heizgas aus einem schweren kohlenstoffhaltigen Einsatzmaterial durch Isolieren eines Teils der Kohlenwasserstoffe aus dem Einsatzmaterial in einer ersten Stufe, Umwandeln des verbleibenden Teils des Einsatzmaterials in Synthesegas in einer zweiten Stufe und Umwandeln des Synthesegases in Kohlenwasserstoffe in einer dritten Stufe, dadurch gekennzeichnet, daß das schwere kohlenstoffhaltige Einsatzmaterial in einer Verkokungszone gecrackt wird, welche ein Bett mit fluidisierten Feststoffen enthält, welche Kohlenwasserstoffe und Koks erzeugen, welcher sich auf den fluidisierten Feststoffen absetzt, daß ein Teil der kokshaltigen Feststoffe in die Vergasungszone eingeführt wird, in welcher mindestens ein Teil des Kokses mit einem sauerstoffhaltigen Gas vergast wird, wodurch eine Kohlenmonoxid und Wasserstoff enthaltende Gasmischung erhalten wird, daß die Kohlenmonoxid und Wasserstoff enthaltende Gasmischung zu 30 bis 70 Volumenprozent, bezogen auf das Volumen von Kohlenmonoxid und Wasserstoff, in Kohlenwasserstoffe in einer Synthesezone umgewandelt wird und daß Heizgas, enthaltend Kohlenmonoxid, Wasserstoff und gasförmige Kohlenwasserstoffe, und mindestens eine flüssige Kohlenwasserstofffraktion aus dem Produkt der Synthesezone abgetrennt werden.

2. Verfahren nach Anspruch 1, in welchem das Crackverfahren ein thermisches Verfahren ist, das bei einer Temperatur von 425 bis 700°C und einem Druck von 1 bis 15 bar durchgeführt wird.

3. Verfahren nach Anspruch 1 oder 2, in welchem die fluidisierten Feststoffe Koksteilchen sind.

4. Verfahren nach einem der Ansprüche 1 bis 3, in welchem die Feststoffe mittels Dampf als fluidisierendem Gas fluidisiert werden.

5. Verfahren nach einem der Ansprüche 1 bis 4, in welchem die in der Verkokungszone hergestellten Kohlenwasserstoffe in mindestens eine Gasfraktion und mindestens eine Flüssigfraktion aufgetrennt werden.

6. Verfahren nach einem der Ansprüche 1 bis 5, in welchem die kokshaltigen Feststoffe, die in der Verkokungszone erhalten worden sind, vor deren Einführung in die Vergasungszone erhitzt werden.

7. Verfahren nach Anspruch 6, in welchem die kokshaltigen Feststoffe mittels eines heißen fluidisierenden Gases und/oder heißer fluidisierter Feststoffe in einem Wirbelbett erhitzt werden.

8. Verfahren nach Anspruch 7, in welchem das heiße fluidisierende Gas das in der Vergasungszone erhaltene Kohlenmonoxid und Wasserstoff enthaltende Gas ist, und daß die heißen fluidisierten Feststoffe solche Feststoffe sind, in welchen der Koks zumindest teilweise in der Vergasungszone umgewandelt worden ist.

9. Verfahren nach einem der Ansprüche 6 bis 8, in welchem die kokshaltigen Feststoffe auf eine zwischen 50 und 150°C über der Temperatur der Verkokungszone liegende Temperatur bei einem Druck von 1 bis 15 bar erhitzt werden.

10. Verfahren nach einem der Ansprüche 1 bis 9, in welchem die Kohlenmonoxid und Wasserstoff enthaltende Gasmischung in der Synthesezone mit einem Kobalt und mindestens ein Metall ausgewählt aus Ruthenium, Zirkonium, Titan und Chrom auf einem Siliciumdioxid, Aluminiumoxid oder Siliciumdioxid-Aluminiumoxid-Träger enthaltenden Katalysator kontaktiert wird.

## Revendications

1. Procédé pour la préparation d'hydrocarbures et de gaz combustible à partir d'une charge de départ carbonée lourde en isolant dans une première étape une partie des hydrocarbures de la charge, en transformant dans une deuxième étape la partie restante de la charge en gaz de synthèse et en transformant dans une troisième étape le gaz de synthèse en hydrocarbures, caractérisé en ce que la charge de départ carbonée lourde est craquée dans une zone de cokéfaction qui contient un lit de particules solides fluidisées, produisant des hydrocarbures et du coke qui se dépose sur les particules solides fluidisées, on introduit une partie des particules solides contenant du coke dans une zone de gazéification dans laquelle au moins une partie du coke est gazéifiée avec un gaz contenant de l'oxygène, donnant un mélange de gaz contenant de l'oxyde de carbone et de l'hydrogène, on transforme le mélange de gaz contenant de l'oxyde de carbone et de l'hydrogène à raison de 30 à 70% en volume, en calculant par rapport au volume d'oxyde de carbone et d'hydrogène, en hydrocarbures dans une zone de synthèse, et on sépare un gaz combustible comprenant de l'oxyde de carbone, de l'hydrogène et des hydrocarbures gazeux et au moins une fraction d'hydrocarbures liquides du produit de la zone de synthèse.

2. Procédé selon la revendication 1, dans lequel le craquage est un craquage thermique effectué à une température comprise entre 425 et 700°C et une pression comprise entre 1 et 15 bars.

3. Procédé selon la revendication 1 ou 2, dans lequel les particules solides fluidisées sont des particules de coke.

4. Procédé selon l'une quelconque des revendications 1—3, dans lequel les particules solides sont fluidisées au moyen de vapeur d'eau comme gaz fluidisant.

5. Procédé selon l'une quelconque des revendications 1—4, dans lequel les hydrocarbures produits dans la zone de cokéfaction sont divisés en au moins une fraction gazeuse et au moins une fraction liquide.

6. Procédé selon l'une quelconque des revendications 1—5, dans lequel les particules solides contenant du coke obtenues en provenance de la zone de cokéfaction sont chauffées avant d'être introduites dans la zone de gazéification.

7. Procédé selon la revendication 6, dans lequel les particules solides contenant du coke sont chauffées dans un lit fluidisé au moyen d'un gaz fluidisant chaud et/ou de particules solides fluidisées.

8. Procédé selon la revendication 7, dans lequel le gaz fluidisant chaud est le gaz contenant de l'oxyde de carbone et de l'hydrogène obtenu dans la zone de gazéification, et les particules solides fluidisées chaudes sont des particules solides dont le coke a été au moins partiellement transformé dans la zone de gazéification.

9. Procédé selon l'une quelconque des revendications 6—8, dans lequel les particules solides contenant du coke sont chauffées à une température comprise entre 50 et 150°C au-dessus de la température de la zone de cokéfaction à une pression comprise entre 1 et 15 bars.

10. Procédé selon l'une quelconque des revendications 1—9, dans lequel dans la zone de synthèse le mélange de gaz contenant de l'oxyde de carbone et de l'hydrogène est mis en contact avec un catalyseur comprenant du cobalt et au moins un métal choisi parmi le ruthénium, le zirconium, le titane et le chrome sur de la silice, de l'alumine ou de la silice-alumine comme support.